# EUROPEAN PATENT SPECIFICATION

(11) **EP 4 236 846 B1**
(45) Date of publication and mention of the grant of the patent: **29.04.2026**
(21) Application number: 21811673.9
(22) Date of filing: 29.10.2021
(51) Int. Cl.: A61B 18/14, A61B 18/00

(54) **ARTHROSCOPIC RESECTION PROBE**
ARTHROSKOPISCHE RESEKTIONSSONDE
SONDE DE RÉSECTION ARTHROSCOPIQUE

(30) Priority: 30.10.2020 US 202063107766 P; 20.07.2021 US 202163223651 P
(43) Date of publication of application: 06.09.2023
(73) Proprietor: Smith & Nephew, Inc., Memphis, TN 38116 (US); Smith & Nephew Orthopaedics AG, 6300 Zug (CH); Smith & Nephew Asia Pacific Pte. Limited, Singapore 138565 (SG)
(72) Inventor: LYNN, Christopher J., Austin, Texas 78735 (US); GOODE, Johnson E., Austin, Texas 78733 (US)
(74) Representative: White, Andrew John
(86) International application number: PCT/US2021/057323
(87) International publication number: WO 2022/094258

(56) References cited:
- WO-A1-2020/172659
- US-A1- 2003 163 126
- US-A1- 2008 188 848
- US-A1- 2015 173 827
- US-A1- 2020 222 108

## Description

### FIELD OF THE INVENTION

This disclosure is related to a surgical apparatus for resecting tissue. This invention is related more particularly to a tissue resecting probe for mechanically resecting and electrosurgically treating tissue.

### BACKGROUND

Electrosurgical systems are used by physicians to perform specific functions during surgical procedures. For example, in an ablation mode, electrosurgical systems use high frequency electrical energy to resect soft tissue such as sinus tissue, adipose tissue or tissue within a joint such as meniscus, or cartilage or synovial tissue. Some electrosurgical systems include aspiration, which may remove the resected tissue during treatment, control the rate of tissue treatment and help to cool the handheld device.

Mechanical cutting tools for tissue resection have also been used for a number of years. These types of tools typically include a powered handpiece and a rotating cutting blade, which is secured in the distal end of the handpiece. In arthroscopic or endoscopic surgery both mechanical resection tools and electrosurgical tools are frequently interchanged within a single cannula, speaking to a need to combine both options in a single device. This may reduce surgery time and complications associated with blood loss. However, combination devices generally tend to come with compromises. For example, at least some of the electrosurgical assembly is preferably located on an external surface of the device, with the inner portion of the device dedicated to the suction and moving mechanical resection components. Reliably securing the electrosurgical assembly with a minimal change in outer profile is challenging. Previous attempted solutions have compromised by provide a probe with a larger profile size, or with reduced function of either mechanical resection or electrosurgical treatment. Therefore, there is a need to provide a combination device that includes a reliably secured electrosurgical assembly construct, while keeping the instrument profile size minimal without compromising function of both mechanical cutting and electrosurgical treatment.

As a further example, mechanical resection tools may include multi-use handles including a motor. These handles and associated components may include metallic, electrically conductive components, more prone to conduct heat. The application of electrosurgical energy may heat the fluid being drawn through the device that may transfer through these handle components. Previous attempted solutions may include adding insulation or less conductive components, which may be bulky, or alternatively adding temperatures sensing, which may interrupt the treatment. Therefore, there is a need provide a combination device that includes a means of limiting the fluid temperatures that are drawn through the device without compromising function of both mechanical cutting and electrosurgical treatment. US2020/222108A1 describes an arthroscopic tissue resecting probe including an elongated shaft having outer and inner sleeves which are formed from an electrically conductive material extending about an axis to a working end. US2008/188848A1A describes a surgical tool arrangement including a powered handpiece which cooperates with a combined electrosurgical and mechanical cutting instrument. US2003/163126A1 describes surgical apparatus and methods which provide enhanced aspiration of gaseous bubbles and reduced drag during placement of the surgical apparatus. US2015/173827A1 describes an electrosurgical device having a tubular outer shaft and an inner shaft.

### NOTATION AND NOMENCLATURE

Certain terms are used throughout the following description and claims to refer to particular system components. As one skilled in the art will appreciate, companies that design and manufacture electrosurgical systems may refer to a component by different names. This document does not intend to distinguish between components that differ in name but not function.

In the following discussion and in the claims, the terms "including" and "comprising" are used in an open-ended fashion, and thus should be interpreted to mean "including, but not limited to... ." Also, the term "couple" or "couples" is intended to mean either an indirect or direct connection. Thus, if a first device couples to a second device, that connection may be through a direct connection or through an indirect connection via other devices and connections.

Reference to a singular item includes the possibility that there are plural of the same items present More specifically, as used herein and in the appended claims, the singular forms "a," "an," "said" and "the" include plural references unless the context clearly dictates otherwise. It is further noted that the claims may be drafted to exclude any optional element. As such, this statement serves as antecedent basis for use of such exclusive terminology as "solely," "only" and the like in connection with the recitation of claim elements, or use of a "negative" limitation. Lastly, it is to be appreciated that unless defined otherwise, all technical and scientific terms used herein have the same meaning as commonly understood by one of ordinary skill in the art to which this invention belongs.

"Ablation" shall mean removal of tissue based on tissue interaction with a plasma.

"Mode of ablation" shall refer to one or more characteristics of an ablation. Lack of ablation (i.e., a lack of plasma) shall not be considered an "ablation mode." A mode which performs only coagulation shall not be considered an "ablation mode."

"Active electrode" shall mean an electrode of disclosed embodiments which produces an electrically-induced tissue-altering effect when brought into contact with, or close proximity to, a tissue targeted for treatment.

"Return electrode" shall mean an electrode of a disclosed embodiment which serves to provide a current flow path for electrical charges with respect to an active electrode, and/or an electrode of a disclosed embodiment which does not itself produce an electrically-induced tissue-altering effect on tissue targeted for treatment.

Where a range of values is provided, it is understood that every intervening value, between the upper and lower limit of that range and any other stated or intervening value in that stated range is encompassed within the invention. Also, it is contemplated that any optional feature of the inventive variations described may be set forth and claimed independently, or in combination with any one or more of the features described herein.

### SUMMARY OF THE INVENTION

The invention provides a tissue resecting probe according to claim 1. Preferred embodiments are defined in the dependent claims.

### BRIEF DESCRIPTION OF THE DRAWINGS

For a detailed description of example embodiments, reference will now be made to the accompanying drawings in which:
FIG. 1 illustrates an example system including a combination resection probe, and associated equipment connected thereto, in accordance with the present disclosure;
FIG. 2 illustrates a blade portion of the combination resection probe, in accordance with the present disclosure;
FIG. 3A illustrates a perspective view of a blade distal portion of the combination resection probe, in accordance with the present disclosure;
FIG. 3B illustrates a bottom view of the blade distal portion illustrated in FIG. 3A accordance with the present disclosure;
FIG. 3C illustrates a side view of the blade distal portion illustrated in FIG. 3A, in accordance with the present disclosure;
FIG. 3D illustrates a cross section view of the blade distal portion illustrated in FIG. 3A, in accordance with the present disclosure;
FIG. 4A illustrates a top view of another embodiment of a blade distal portion of the combination resection probe, in accordance with the present disclosure;
FIG. 4B illustrates a side view of the blade distal portion in FIG. 4A, in accordance with the present disclosure;
FIG. 5A illustrates a perspective view of another embodiment of a blade distal portion of the combination resection probe, in accordance with the present disclosure;
FIG. 5B illustrates an exploded view of the blade distal portion illustrated in FIG. 5A in accordance with the present disclosure;
FIG. 5C illustrates another view of the blade distal portion illustrated in FIG. 5A, in accordance with the present disclosure;
FIG. 5D illustrates a side view of the blade distal portion illustrated in FIG. 5A, in accordance with the present disclosure;
FIG. 5E illustrates an end view of the blade distal portion illustrated in FIG. 5A, in accordance with the present disclosure;

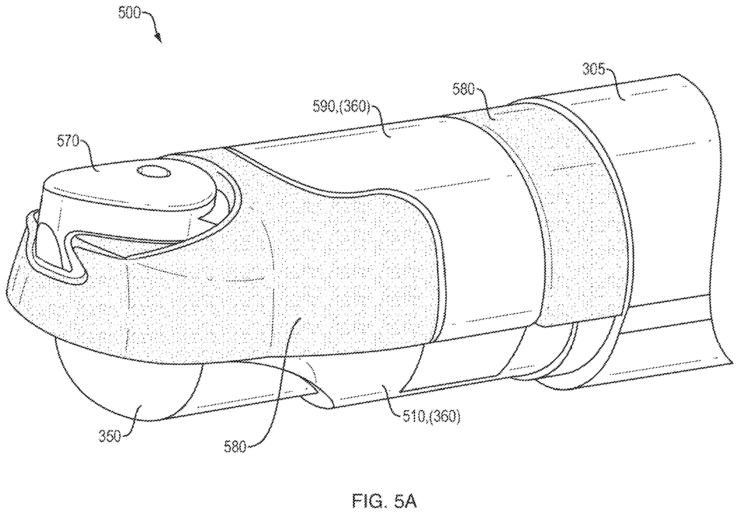

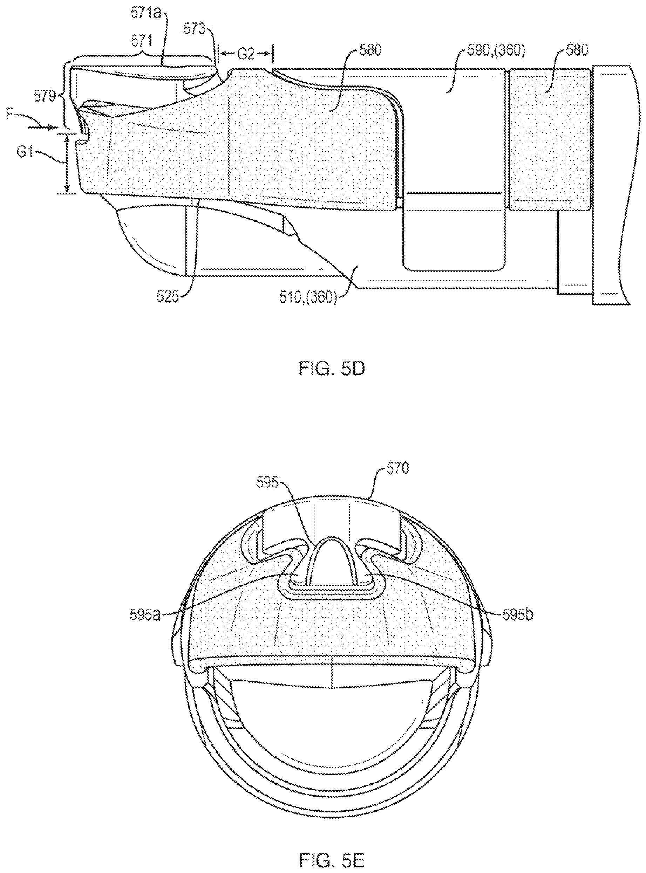

FIG. 5E illustrates another exploded view of the blade distal portion illustrated in FIG. 5A, in accordance with the present disclosure;

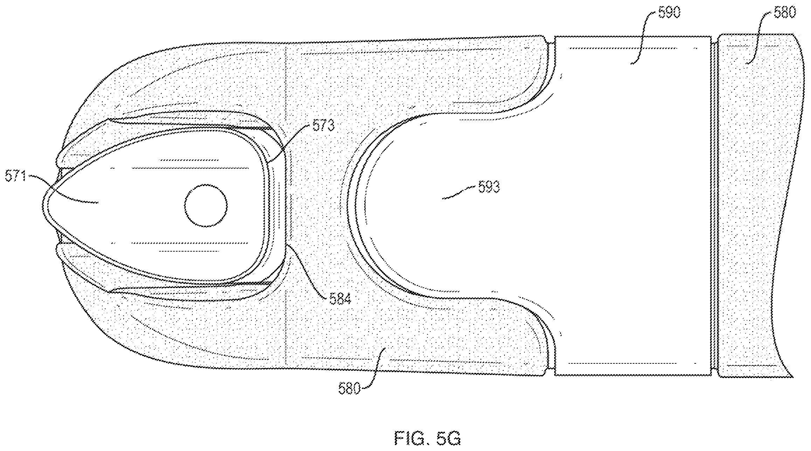

FIG. 5G illustrates a top view of the blade distal portion illustrated in FIG. 5A, in accordance with the present disclosure;

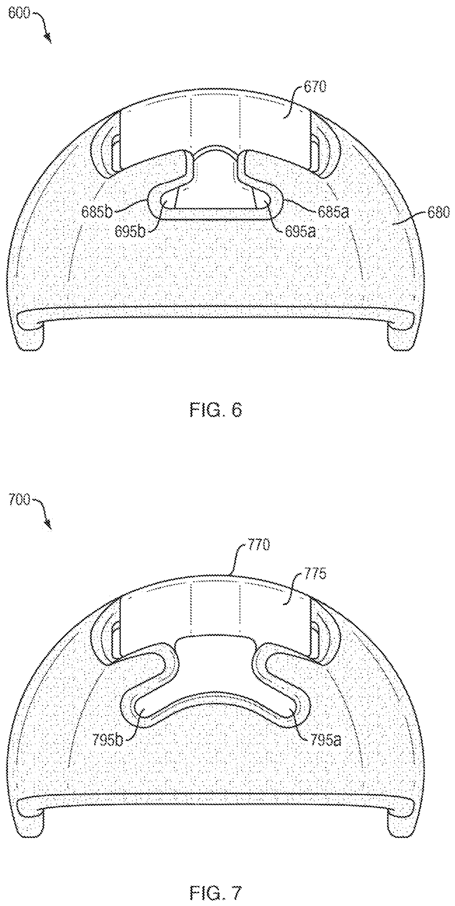

FIG. 6 illustrates an end view of another embodiment of a blade distal portion of the combination resection probe, in accordance with the present disclosure;
FIG. 7 illustrates an end view of another embodiment of a blade distal portion of the combination resection probe, in accordance with the present disclosure;

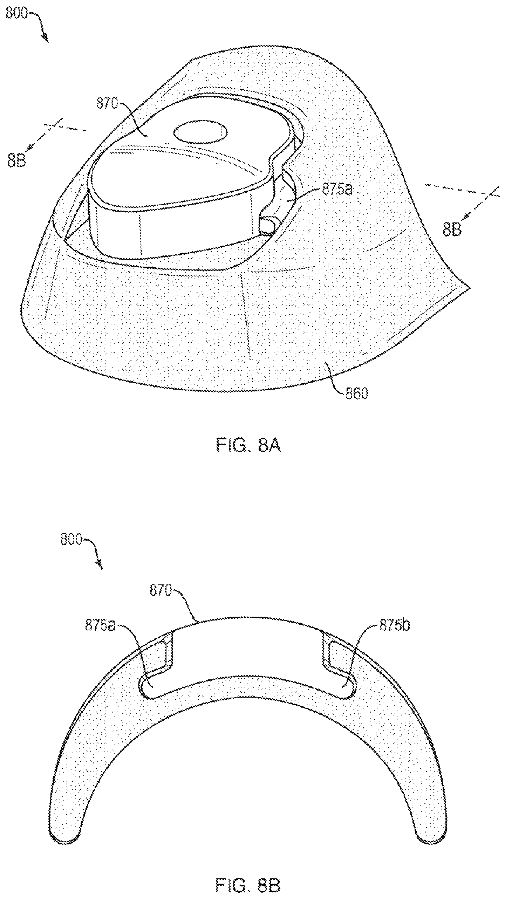

FIG. 8A illustrates a perspective view of another embodiment of a blade distal end of the combination resection probe, in accordance with the present disclosure;
FIG. 8B illustrates a cross section view of the blade distal portion illustrated in FIG. 8A in accordance with the present disclosure;

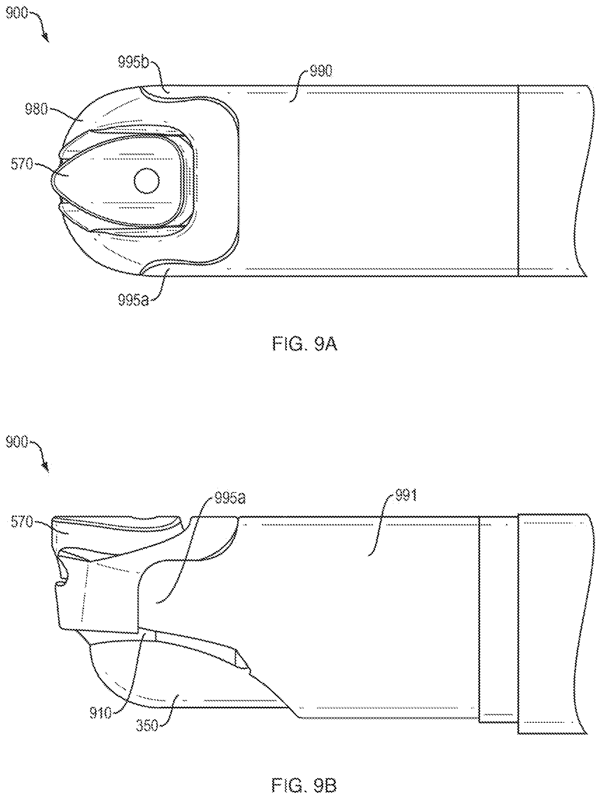

FIG. 9A illustrates a top view of another embodiment of a blade distal end of the combination resection probe, in accordance with the present disclosure;
FIG. 9B illustrates a side view of the blade distal portion illustrated in FIG. 9A in accordance with the present disclosure;

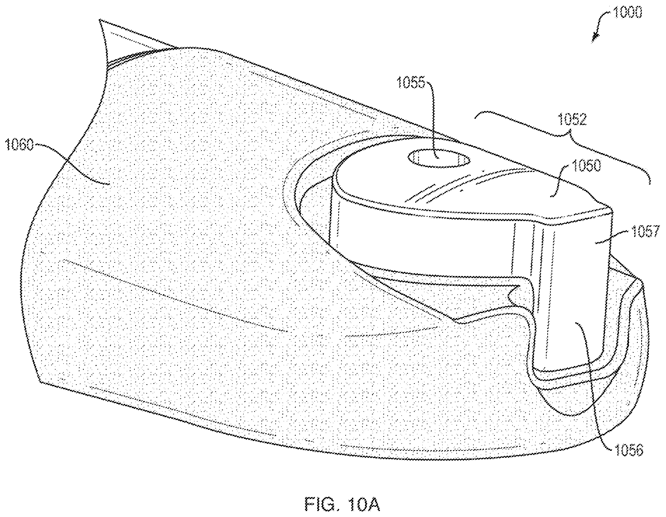

FIG. 10A illustrates a perspective view of another embodiment of a blade distal end of the combination resection probe, in accordance with the present disclosure;

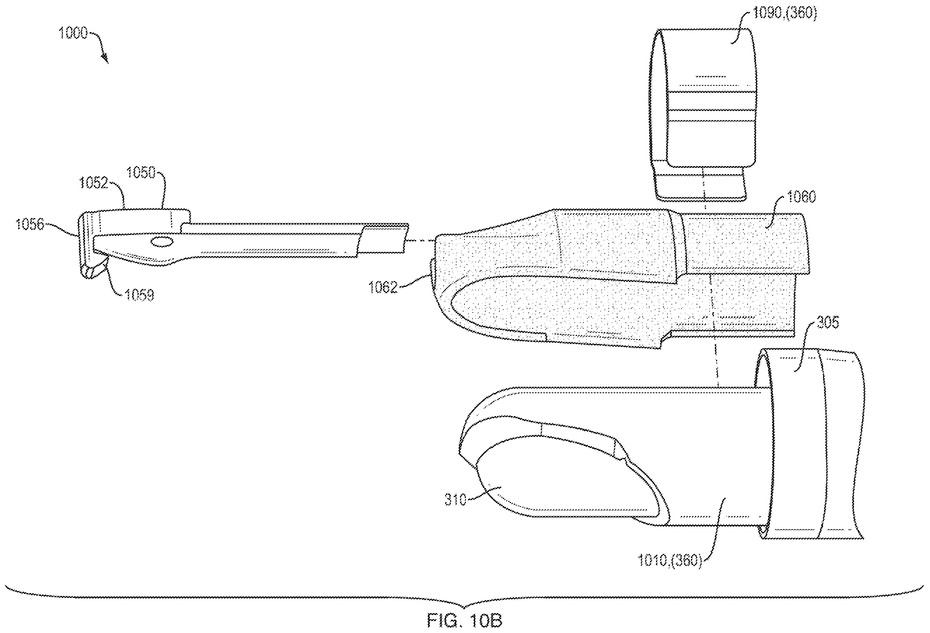

FIG. 10B illustrates an exploded view of the blade distal portion illustrated in FIG. 10A, in accordance with the present disclosure;

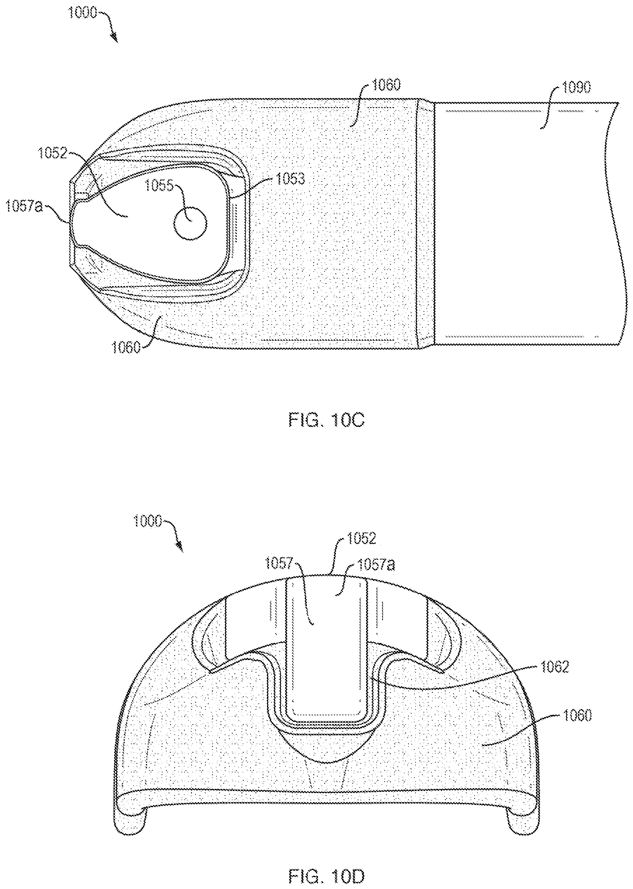

FIG. 10C illustrates a top view of the blade distal portion illustrated in FIG. 10A, in accordance with the present disclosure;
FIG. 10D illustrates an end view of the blade distal portion illustrated in FIG. 10A, in accordance with the present disclosure; and

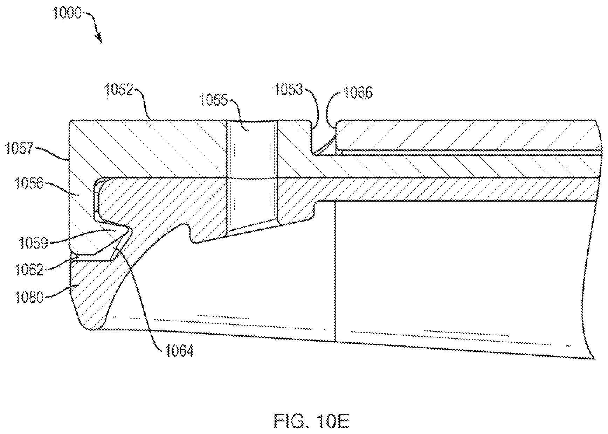

FIG. 10E illustrates a cross section view of the blade distal portion illustrated in FIG. 10A, in accordance with the present disclosure.

### SUMMARY

Generally this disclosure describes a system including a combination device that both mechanically resects tissue and electrosurgically treats tissue. Various embodiments are therefore directed to a variety of systems for removing tissue, using mechanical cutting, electrosurgical ablation and aspiration. The specification now turns to an example system.

Various embodiments are directed to a combination arthroscopic probe for removing and treating tissue in a patient, including a handle portion and a blade portion. The handle portion includes a motor that is selectively coupled to a control box for controlling the motor. The blade portion includes an outer sleeve with a lumen extending therethrough and an inner shaft along the lumen. The outer sleeve may be fixed to the handle portion as a fixed or stationary sleeve. The outer sleeve has a window at the distal end, the window having a cutting edge. The inner shaft may also have a cutting edge at its distal end and is coupled to the motor, operable to cause the inner shaft to move and in cooperation with the outer sleeve window cutting edge mechanically cut tissue. The inner shaft may rotate. The inner shaft may define a window, axially coincident with the outer sleeve window. The inner shaft may include an elongate bore, in communication with an aspiration apparatus, configured to remove fluids and resection by-products from the treatment site through the inner shaft window and the along the inner shaft.

The outer sleeve comprises an electrically conductive material that is at least partially exposed defining an exposed electrically conductive portion at the distal end of outer sleeve. The outer sleeve may carry a dielectric spacer that may define a portion of the outer sleeve inner lumen. The dielectric spacer may carry an active electrode and electrically isolates the active electrode from the outer sleeve. Dielectric spacer may be formed of any electrically insulative material that may is preferably resistant to degradation from plasma. Dielectric spacer may be formed of a ceramic. Dielectric spacer is configured to electrically insulate active electrode from exposed electrically conductive portion of sleeve, such that sleeve may be electrically coupled to an RF generator and operate as a return electrode.

An example tissue resecting probe is disclosed for mechanically resecting and electrosurgically treating tissue. The probe has a tubular distal end that is electrically conductive, with a cutting window on a first circumferential side and an electrically insulative spacer wrapped over a second circumferential side. An active electrode is secured to the electrically insulative spacer. The active electrode has two portions, that are angularly offset from each other. The first of these portions extends circumferentially and axially along the tubular distal end. The second of these portions extends from a distal end of the first portion and across the tubular distal end. The second portion has at least one flange that engages a corresponding notch in the electrically insulative spacer to resist separation of the active electrode from the electrically insulative spacer.

In some particular embodiments, the second portion extends perpendicularly to the longitudinal axis. The second portion may define a distal-most surface of the tissue resecting probe. The first portion may be is axially tapered, to finely dissect tissue at the first portion distal end. In some examples, embodiments, the at least one flange may extend proximally into the electrically insulative spacer or the at least one flange may include bilateral flanges. The second portion may define a distal facing concave surface that helps to deflect external forces on the active electrode during use. In some example embodiments, the at least one flange may to engage the notch and concomitantly axially position the active electrode to maintain an axial gap between the insulative spacer and a proximal end of the active electrode, the axial gap configured to mitigate stress concentrations along the tubular distal end during use. In some example embodiments, the insulative spacer may extend around the tubular distal end up to a cutting edge of the cutting window. The insulative spacer may extend over and cover a distal facing end surface of the tubular distal end, thereby defining at least a portion of a distal-most end of the tissue resecting probe. The active electrode may include a neck extending proximally from the active electrode first portion configured to extend along a tunnel through the insulative spacer and thereby retain the active electrode at a second location proximally separated from the second portion. In some example embodiments, the tissue resecting probe may include a retention clip that extends around an external surface of insulative spacer and both electrically and mechanically couples to the tubular distal end. The tubular distal end and retention clip may both define external surfaces of the tissue resecting probe. The retention clip may include a distally extending tab on a same circumferential side as the active electrode, this tab configured to arrange a proximal end of the active electrode and the retention clip according to a target spacing and thereby improve the electrosurgical tissue effect. The retention clip may include bilateral radially jogged ends.

Another example embodiment tissue resecting probe is disclosed for mechanically resecting and electrosurgically treating tissue. This probe includes a tubular distal end that defines a return electrode. An aperture with a cutting edge is disposed on a first circumferential side of the tubular distal end. An insulator spacer is wrapped around a second circumferential side of the tubular distal end opposite the aperture. An active electrode is secured to the insulative spacer, the active electrode having a first portion extending axially and circumferentially along the tubular distal end and a retention hook extending from a distal end of the first portion and angularly offset therefrom. The retention hook is configured to attach a distal end of the active electrode with the insulative spacer and resist separation of the active electrode from the insulative spacer.

In some particular embodiments, the retention hook may define a distal facing surface of the tissue resecting probe. The retention hook may include a first projection that extends towards a longitudinal axis of the tissue resecting probe and flange projection extending from the first projection, configured to fit within a notch at a distal end of the insulative spacer. The flange projection may extend along the longitudinal axis.

A tubular end effector of a combination electrosurgical and mechanical resection assembly is also disclosed herein. The end effector includes a metal component defining a cutting window on a first circumferential side of the tubular end effector. The metal component defines a return electrode of the assembly. The end effector also includes a ceramic spacer attached to the metal component such that the ceramic spacer forms a second circumferential side of the tubular end effector opposing the first circumferential side. A distal end surface of the ceramic spacer includes a groove. The tubular end effector also includes an active electrode secured to the ceramic spacer, the active electrode positioned opposite the cutting window. The active electrode includes a circumferential treatment surface and a retention hook extending from a distal end of the circumferential treatment surface and angularly offset therefrom. The retention hook includes at least one projection for operatively coupling within the groove and thereby retaining the active electrode with the tubular end effector.

Another example embodiment is disclosed herein of an arthroscopic tissue resecting probe may include an elongated shaft with an outer, electrically conductive sleeve and an inner sleeve. Each sleeve includes a distal region defining a cutting window and the inner sleeve is rotatable to cut tissue. The probe may also include a ceramic body disposed on an outer surface of the outer sleeve along the distal region of the outer sleeve. An electrode is carried by the ceramic body and a first aspiration opening extends through the electrode, the ceramic body and the outer sleeve for aspirating fluid and ablation by-products therethrough and into an interior of the shaft. A second aspiration opening is defined at least partially by the electrically conductive sleeve and spaced away from the electrode for aspiration therethrough to the interior of the shaft.

In some example embodiments of the arthroscopic tissue resecting probe the electrode, ceramic body and outer sleeve all include a 360-degree bounded hole spaced from the outer sleeve cutting window defining the first aspiration opening. The bounded holes are aligned for aspiration through the bounded holes to the interior of the shaft, the bounded holes being aligned along an axis transverse to a longitudinal axis of the elongated shaft. In some example embodiments of the probe, the electrically conductive sleeve and inner sleeve both define a 360-degree bounded hole both spaced away from the electrode and when aligned form the second aspiration opening. Alternatively, the second aspiration opening may be defined by boundaries defined by the cutting edges of both sleeves. The second aspiration opening may be proximally disposed relative to the electrode. The second aspiration opening may be proximally disposed relative to the first aspiration opening. The second aspiration opening may be proximally disposed relative to both cutting windows. The first and second aspiration openings may each have an opening size configured to balance aspiration through the first aspiration opening at a flow rate that forms a stable plasma at the electrode. The second aspiration opening may be configured to receive fluid therethrough, to cool the fluid aspirated through the first aspiration opening and thereby mitigate heating of the probe.

In a further example embodiment disclosed herein, an arthroscopic tissue resecting probe may include an elongated shaft with an outer, electrically conductive sleeve and an inner sleeve, each sleeve having a distal region defining a cutting window, the inner sleeve being rotatable to cut tissue. A ceramic body is disposed along an outer surface of the outer sleeve on the distal region of the outer sleeve. An electrode is carried by the ceramic body, and a first aspiration opening extends through the electrode, the ceramic body and the outer sleeve for aspirating fluid and ablation by-products therethrough and into an interior of the shaft. The probe also includes a clip or collar that covers and secures a portion of the ceramic body to the distal region, the clip or collar separately formed and directly fixed to the electrically conductive sleeve,

In some example embodiments the collar or clip circumferentially sandwiches the ceramic body between the electrically conductive sleeve and collar or clip, The collar or clip may be proximally spaced from the cutting windows. The collar or clip may define a thin band that fully wraps around the entire electrically conductive sleeve. The collar or clip may be electrically conductive and be in electrical communication with the electrically conductive sleeve.

### DETAILED DESCRIPTION

The following discussion is directed to various embodiments. Although one or more of these embodiments may be preferred, the embodiments disclosed should not be interpreted, or otherwise used, as limiting the scope of the disclosure, including the claims. In addition, one skilled in the art will understand that the following description has broad application, and the discussion of any embodiment is meant only to be exemplary of that embodiment, and not intended to intimate that the scope of the disclosure, including the claims, is limited to that embodiment.

The disclosure may generally include a system that combines mechanical resection and electrosurgical tissue treatments in a single device, electrosurgical tissue treatment including but not limited to tissue ablation, cutting, shrinking and coagulation. Combining these two modalities may reduce the need for multiple instruments during surgery, such as endoscopic or arthroscopic surgery. The mechanical blade edge surfaces of both the stationary member and rotating member preferably comprises a metal. The active and return electrodes are preferably both disposed on a stationary outer sleeve of the blade. A stationary sleeve may make the electrical communication between the energy RF energy source and electrodes, According to the invention, the active electrode extends up to and around a distal-most surface of the device, which improves accessibility of the active electrode to the target tissue and thereby improves the electrosurgical treatment to the target tissue. The electrodes are also integrated with or form a portion of the outer sleeve portion and are configured to maintain a low-profile device distal end, minimally altering in outer diameter by the addition of the electrosurgical assembly.

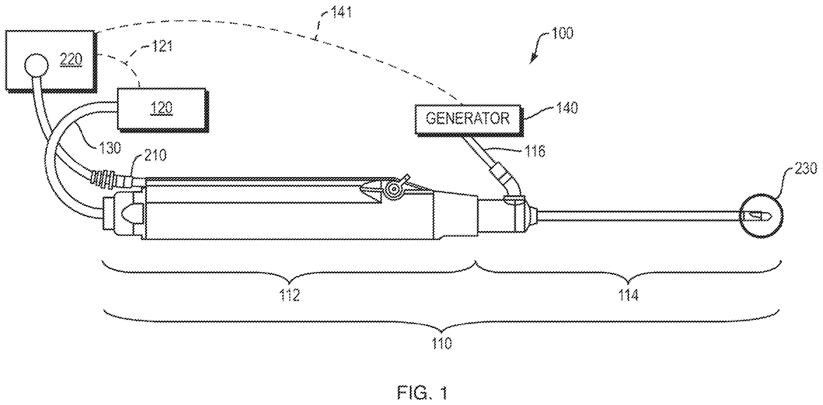

An overview of an example surgical system 100 is illustrated in FIG. 1 and includes a combination arthroscopic resecting device 110 having a handle portion that includes a motor drive unit ("MDU") 112. Handle portion may be removably coupled to a blade 114. MDU 112 may be operatively coupled to a controller/power supply 120 via cord 130. Actuation of the MDU 112 moves a portion of the blade 114 relative to another portion of the blade to resect tissue. Combination device 110 may be configured to mechanically resect tissue in a manner similar to commercially available metal shavers or burrs with high speed rotating inner shafts coupled to the MDU, disposed within a stationary outer sleeve. System 100 also includes an RF generator 140 for supplying RF power to blade 114 via RF power cord 116. In some embodiments the controller/power supply 120 and RF generator 140 may be in a single enclosure. In some embodiments the controller/power supply 120 and RF generator 140 may be in communication with each other. In some embodiments, at one of the controller/power supply 120 and RF generator 140 may be in communication with (121, 141) flow control apparatus 220.

Combination arthroscopic resecting device 110 includes an aspiration tube 210 that may extend along the MDU 112 and may couple to an aspiration control apparatus 220. Aspiration control apparatus 220 may control a fluid flow or negative pressure through tube 210 and may include a suction pump or plurality of suction pumps (not shown). Aspiration control apparatus 220 may be a peristaltic pump having a rotor to provide and control aspiration of tissue and fluid from the target site and along device 110. In some embodiments, aspiration may be fluidly coupled to wall suction that draws a vacuum at a consistent level. Aspiration control apparatus 220 may include a pinch valve having a plurality of positions that pinch or release the aspiration tube or alternative means of altering a fluid conduit size (orifice) associated with the aspiration tube. In some embodiments, aspiration may be at least partially controlled by a control valve disposed on the MDU 112 that may selectively control a value associated with aspiration through the device. Blade 114 includes at least one lumen that extends through blade 114 and communicates with aspiration tube 210 for aspiration at blade distal portion 230, such that fluid and debris may be aspirated through a window in blade distal portion 230, flowing along lumen and through tube 210.

Controller/power supply 120 may be, for example, the Dyonics^{®} Power Shaver system or the Dyonics^{®} EP-18 Shaver System supplied by Smith & Nephew, Inc. of Andover, Mass. Generator 140 may be a commercially available generator such as, for example, a COBLATION WEREWOLF system supplied by Smith and Nephew, Inc of Andover, Mass. The blade 114 is sized to accommodate the desired application. For example, for use in a shoulder the blade may be sized differently from one for use in a prostate, Applications include, for example, use in a shoulder, a knee, and other joints, as well as use in natural orifices such as, for example, a uterus, a urethra, a nasal cavity, and a mouth. MDU 112 includes a drive shaft (not shown) that is configured to selectively couple with a proximal end of blade 114, and may include coupling means similar to those disclosed in commonly assigned U.S. Patent No. 7,150,747**.**

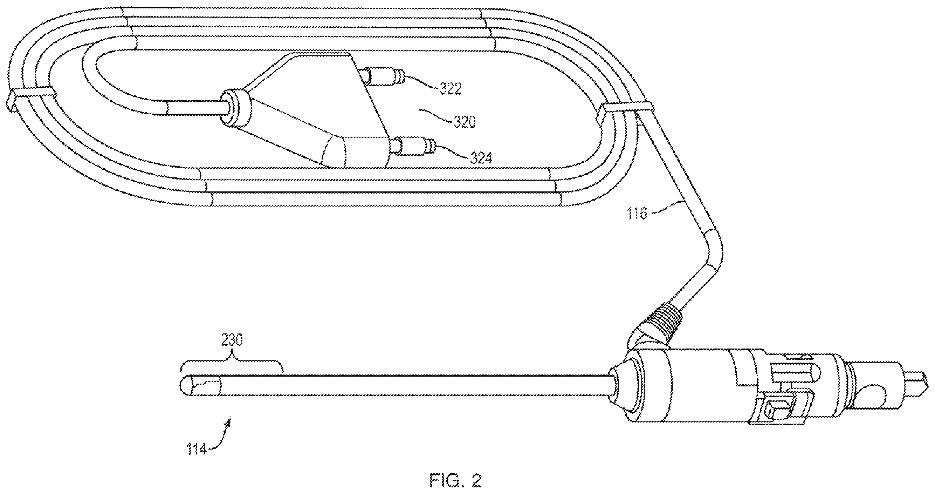

Referring to FIG. 2, an example blade 114 is shown, including a RF power cord 116 terminating at a free end with a connector 320. Connector 320 may have at least two prongs 322 and 324 designed to connect to either a generator 140 or a foot switch. Each prong connects to one of two separate conductors within RF power cord 116 to provide both a supply path and a return path for blade 114. Additional prongs (not shown) may provide further information to the RF generator/controller 140 such as the type of device and/or desired or candidate settings such as fluid flow settings or power settings. Alternatively, connector may include a series of pins configured to connect to a connector of an RF generator 140, such as the system described in the commonly assigned U.S. Patent No. 9,333,024.

The blade 114 mechanically may resect tissue in a manner similar to other commercially available mechanical resections devices. For example, blade 114 may include at least two elongate sleeves or tubes, concentrically arranged, one inside the other. One of the elongate tubes defines an outer sleeve that may be fixedly attached to the MDU 112 and is a stationary sleeve during use. Another of the sleeves is defined as an inner sleeve, concentric with the outer sleeve, coupled to the MDU 112 such that it may rotate at high speeds relative to the stationary outer sleeve. Blade 114 can be attached to MDU 112 using various hub style structures, for example, threaded connections and pressure-fit connections. Various embodiments of MDU 112 include motor drive units manufactured by Smith & Nephew, Inc., of Andover, Mass., such as, for example, part numbers 7205354, 7205355, and 7205971. A variety of cutting surfaces can provide mechanical cutting. Such surfaces include, for example, blades that are curved, burred, straight, serrated, or miniature. Mechanical cutting is typically achieved with speeds in the thousands of cycles (for example, revolutions or reciprocations) per minute.

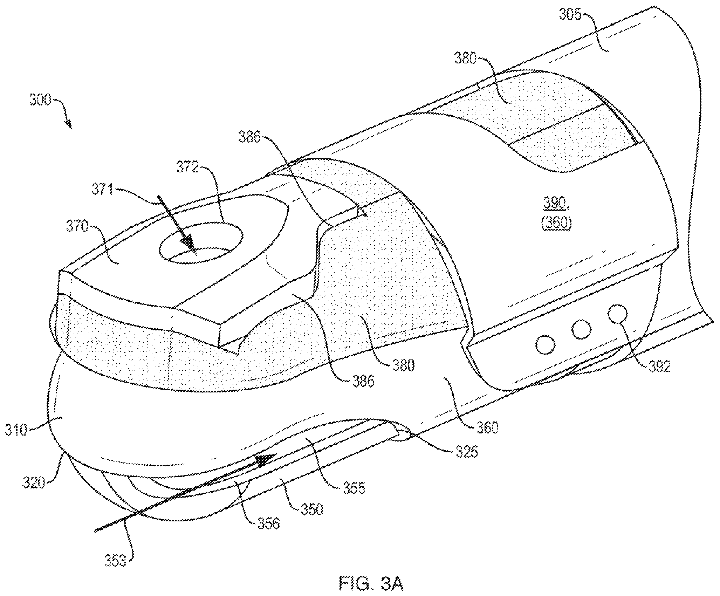

Now turning to FIGS. 3A-3D, where an example embodiment of the distal end 300 of blade 114 is illustrated. Blade distal end 300 includes both a means to both mechanically and electrosurgically treat tissue. Blade distal end 300 is generally tubular is shape and includes an outer sleeve 310 with a window 320 on a first circumferential side of the sleeve 310. Window 320 has an edge surface 325 around the perimeter of the window 320. At least a portion of the edge surface 325 is sufficiently sharp to cut tissue when used in combination with the inner sleeve 350. Inner sleeve 350 may extend along a lumen or bore of outer sleeve 310 and may have a similar window 355 and edge surface 356 that is configured to cooperate with edge surface 325 to mechanically resect tissue as the inner sleeve rotates.

Outer sleeve 310 is preferably a metallic and electrically conductive tube, configured to provide an electrical path from the electrical cord 116 and along the sleeve 310. Outer sleeve 310 may be at least partially coated or covered by a layer or sheath 305 to electrically insulate a portion of the outer sleeve 310 and limit an exposed portion of the outer sleeve to controlled areas. For example, a proximal portion of the outer sleeve 310, adjacent the MDU 112 may be exposed, sufficient to electrically couple with cord 116 and thereby the RF generator 140 (not shown). A distal portion of the outer sleeve 310 may also be exposed, exposing the metallic edge surface 325 for mechanical resection. The exposed surface area (non-coated or covered with insulating material) of the outer sleeve 310 defines a return electrode 360 of the electrosurgical assembly.

Outer sleeve 310 carries an insulative spacer 380 and an active electrode 370. Active electrode 370 has a rounded outer surface and nests at least partially within a spacer recess 386, minimizing any additional size increase so the device may fit within a 5mm cannula. Active electrode 370 is electrically coupled to the RF generator 140 via cables or wires extending along the blade 114 (not shown) and connector 320. Active electrode 370 and associated cables are preferably electrically insulated from the outer sleeve 310, allowing the outer sleeve 310 to act as the return electrode 360. Active electrode 370 is electrically isolated from outer sleeve 310/return electrode 360 via electrically insulative spacer 380. Since the active electrode 370 is intended to selectively ablate tissue and therefore form plasma thereon, spacer 380 is preferably an electrically insulative material that is also resistant to degradation from plasma, or plasma-hardy. Materials may include a ceramic or glass material, such as alumina, zirconia and the like.

The active electrode 370 should preferably be a material that is resistant to degradation to plasma, such as a tungsten, titanium, platinum, molybdenum, aluminum, gold and copper. More specifically the active electrode 370 may be a different material from the inner and outer sleeve material. While stainless steel is preferred for the mechanical cutting edges, stainless steel tends to be less resistant to degradation by plasma and therefore is not the preferred material for an ablation electrode 370. Tungsten however is a more brittle metal than stainless steel and therefore would not be preferable as a cutting edge, as particulate may form during mechanical resection. Additionally, the inventors have found that the distance between edges or periphery of the active electrode 370 and cutting edge 325 should be at or above a predetermined distance. This predetermined distance is configured to inhibit inadvertent plasma formation on the cutting edge 325. If closer than this predetermined distance, not only may plasma form along the cutting edge 325 cause inadvertently tissue treatment on tissues other than the target tissue, but this plasma may dull or degrade the cutting edge 325, potentially frustrating mechanical resection. A minimum distance between peripheral edges of the active electrode 370 and the cutting edge 325 should be at least 2mm measured along the outer surface of the device.

To minimize any addition diameter to the distal end 300, due to the electrosurgical assembly, spacer 380 is a thin component that circumferentially engages an outer surface of the sleeve 310. Spacer 380 inner surface is contoured to mate with outer surface of sleeve 310 and may be attached using adhesive for example. Spacer 370 is preferably formed from a high-strength ceramic which provides structural integrity to the outer sleeve 310 and may add rigidity to the outer sleeve. Spacer 380 may be secured using adhesive and therefore a larger mating surface area for adhesion is preferable for a higher level of hold between the two components. As illustrated throughout FIGS. 3A-3D, spacer 380 may extend proximally along outer sleeve 310 up to at least sheath 305. While this proximal extension may improve securement of the spacer 380, this also insulates a proximal side of the return electrode 360. This may result in a more inert proximal side of the active electrode, less likely to form plasma. Distal end 300 therefore may include a clip or collar 390 annularly disposed around the spacer 380. Clip 390 may be electrically conductive and in electrical communication with outer sleeve 310 and therefore form a portion of return electrode 360. Clip 390 may be welded 392 to outer sleeve 310. This therefore resumes a target proximity of the return electrode 360 adjacent the proximal side of the active electrode 370. Clip 390 may also increase securement between the sleeve 310 and spacer 380. Spacer 370 may include an annular notch, configured to receive the clip 390 therein, while minimally increasing an outer diameter of distal end 300.

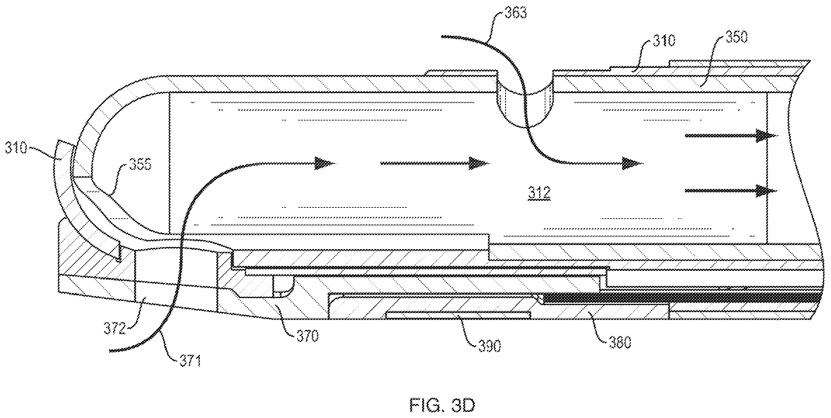

Best illustrated in FIG. 3D, inner sleeve 350 may define an elongate fluid aspiration conduit 312 therealong, in fluid communication with aspiration tube 210, so that tissue debris formed during mechanical resection may be removed through windows 320, 355 and along conduit 312. A second entrance 371 into the conduit 312 is formed via at least one spacer aperture (seen best in FIG. 3D) aligned with an active electrode aperture 372. The electrode 370, the ceramic spacer 380 and the outer sleeve 310 each define an aspiration aperture that may define a 360-degree bounded aperture. Second entrance 371, defined by these apertures may be spaced from the outer sleeve cutting window 320, the holes being aligned for aspiration through to the interior of the shaft and thereby conduit 312. The plurality of apertures may be aligned along an axis transverse to a longitudinal axis of the elongated shaft. During electrosurgical treatment, the inner sleeve 350 may be stationary and aligned to at least partially face this second entrance 371. An example of this is disclosed in more detail in International Patent Application No. PCT 2020/019479 filed February 24, 2020, titled "COMBINATION ELECTROSURGICAL AND MECHANICAL RESECTION DEVICE", commonly owned. Second entrance is configured to draw fluid cross the active electrode 370 and remove fluid, debris and plasma by-products through the active electrode surface during use. Control of a value associated with the aspiration flow rate may also adjust the electrosurgical tissue effect, also disclosed in International Patent Application No. PCT 2020/019479 filed February 24, 2020, titled "COMBINATION ELECTROSURGICAL AND MECHANICAL RESECTION DEVICE", commonly owned.

Distal end 300 may include a means to regulate a temperature of the device, and in particular the external surfaces of the device such as the external surface of the MDU 112. When fluid is drawn through the second entrance, while supplying electrosurgical energy to the active electrode 370, fluid temperatures may be elevated. This elevated temperature may increase temperatures along the device surfaces including the handle and fluid lines of the device. The surface area of each of these components must stay under the touch temperatures specified in IEC 60601-1 standard. Since the MDU 112 is typically reusable, it may be formed of a metal material, such as aluminum, typically more thermally conductive than for example, a disposable electrosurgical device that is predominately formed of polymers. One embodiment may include a temperature sensor along the blade 114, that upon detection of a predetermined temperature increase, may communicate with the RF generator. This may trigger and indication warning to the user or alter the RF energy output. Temperature sensors may be similar to those disclosed in at least US. Patent 8,355,799; 8,696,659; 9,452,008; or 10,420,601 commonly owned.

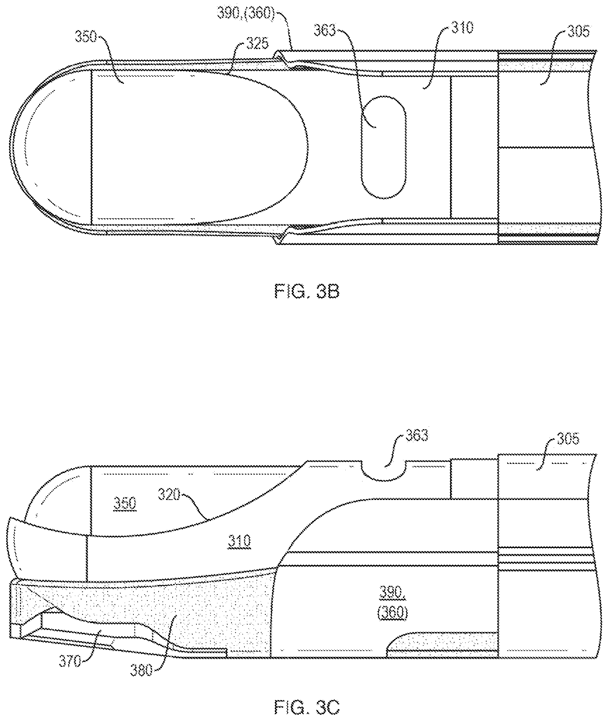

In some embodiments, to alleviate the elevated temperatures, cooler fluid may be routed through the device. This may regulate the temperature of the aspirated fluid through the device lumen 312 and then through the MDU 112. The distal end 300 may include a bypass aspiration pathway 363 entirely spaced away from the active electrode 370 to route cooler fluid into and along the lumen 312. Pathway 363 may extend through an opening in both the outer and inner sleeves (310, 350), and provides a route to draw a portion of fluid from within the joint cavity into the fluid aspiration conduit 312. This portion of fluid may be relatively cooler than fluid that is aspirated through the entrance 371, and thereby through the active electrode aperture 372. To some extent, depending on at least the power setting of the RF generator 140 and rate of fluid aspiration, the aspirated fluid and by-products through the second entrance 371 may have an elevated temperature as a result of passing over the active electrode 370. Over time, this elevated temperature may increase external surface temperatures of the MDU 112 and tubing 210. The secondary pathway 363 may mix cooler fluids with these elevated temperatures and help cool this fluid and thereby mitigate increasing external surface temperatures. Shown in FIG. 3B, 3C and 3D, a bypass pathway 363 may be spaced proximally from the active electrode 370. Bypass pathway 363 may be disposed on an opposing circumferential side than the active electrode 360. Being spaced away from the active electrode 370 preferably draws fluid having a temperature less influenced by the active electrode energy into the fluid aspiration conduit 312. Bypass entrance 363 is defined by a hole in the inner sleeve 350 and a hole though the outer sleeve 310, that overlap. Each hole may define a 360-degree bounded hole or aperture.

Returning to FIG. 3A, illustrated in another optional bypass entrance 353 (in addition to or instead of bypass entrance 363), defined by the relative locations between the two windows (320, 355). This pathway 353 is adjustable, and the inner sleeve 350 may be rotated to a position to adjust the aperture entrance size, should temperatures of the MDU be elevated. For example, a temperature sensor located along the device 110 may sense a value indicative of an elevated temperature, the temperature sensor in communication with the MDU 112. MDU 112 may then rotate inner sleeve 350 to a position that forms a bypass entrance 353. Upon the temperature sensor sensing a value indicative that temperature has decreased to a predetermined value, the communication between the sensor and MDU 112 may be configured to return the inner sleeve 350 to a fully closed configuration. Upon the temperature sensor sensing a value indicative that temperature has increased or remained at the elevated value, the communication between the sensor and MDU 112 may be configured to increase the entrance size by moving the inner sleeve 350 to a different position. In alternative embodiments, not shown, the bypass pathway may include a plurality of pathways spaced away from the active electrode 360. Alternatively, a bypass pathway may include at least one aperture solely through the inner sleeve 350 (not shown), axially opposite the inner sleeve window 355, and thereby axially opposite the active electrode 370.

Flow associated with the bypass entrance(s) 363, 371 may also regulate the tissue effect and energy within any plasma formed at the active electrode 370. Combination devices may be fluidly coupled to wall suction, which may have a high aspiration flow rate that may be too strong a flow for plasma to stably form at the active electrode 370. Too high a flow may extinguish or destabilize any plasma formed at the active electrode 370. Therefore, a bypass entrance(s) 363, 371 may balance or govern the fluid flow rate through the electrode aperture 372 and thereby maintain a more stable plasma at the active electrode 370. For example, the RF generator may sense a value indicative of plasma stability, as disclosed in at least US. Patent 8,192,424 and 10,448,988, commonly owned. RF generator may be in communication with MDU 112, such that upon sensing a value associated with non-target plasma stability, the inner sleeve 350 may be moved to a position that forms a bypass entrance 371 and adjusts the flow of fluid through aperture 372, and thereby may the plasma stability.

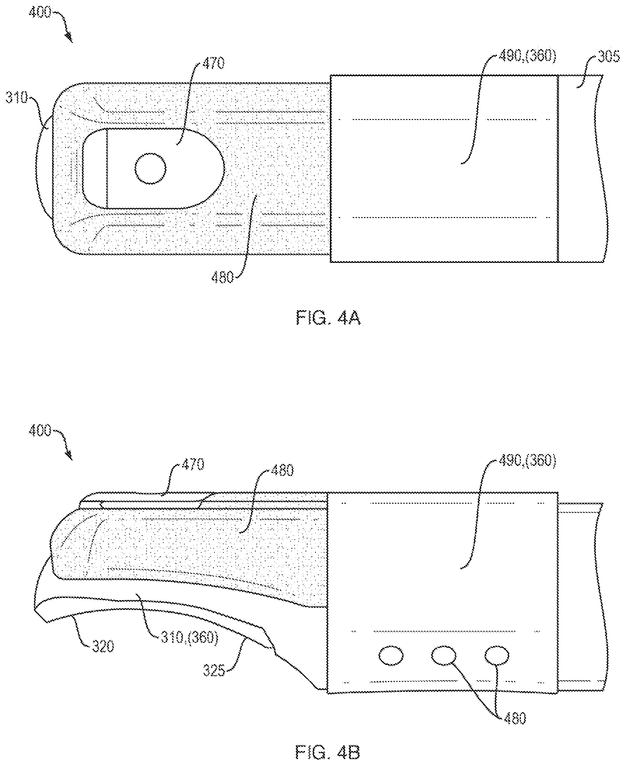

Turning now to FIG. 4A and 4B, views of another embodiment of a distal end 400 are illustrated with the inner sleeve removed for clarity of description. Similar elements are given the same numerical indicator as distal end 300. Distal end 400 may include an outer sleeve 310 carrying an insulative spacer 480 that carries an active electrode 470. In order to maintain a minimal outer diameter of the device distal end 400, a thin spacer 480 circumferentially wraps around a portion of the outer sleeve outer surface. Spacer 480 is configured to electrically isolate the active electrode 470 from the outer sleeve 310, the outer sleeve 310 electrically connected as a return electrode, as disclosed herein. Spacer 470 wraps around the outer surface of the outer sleeve for a limited distance to define an outermost boundary along and around the outer sleeve 310, this boundary exposing sufficient surface portion of the outer sleeve 310 around the active electrode 470, the exposed surface portion defining the surface area of the return electrode 360. To maintain a minimal cross section profile, spacer 480 is a thin component. Spacer 480 may be approximately 0.010 inches thick. Spacer 480 inner surface may mate with the outer surface of the sleeve 310 and be adhesively coupled to the outer sleeve 310. As disclosed previously herein, when it comes to having a strong bond between the sleeve 310 and spacer 480, a larger area of contact mating the two is preferable. Therefore, the spacer 480 preferably extends circumferentially around the sleeve 310 and proximally along the sleeve 310 from the active electrode 470 substantially. Stated in another way, the spacer 470 may extend further around and along the sleeve 310 than the minimum necessary for electrical isolation between the sleeve 310 and active electrode 470, to increase securement between the sleeve and spacer 480.

With this bias towards securement however, the electrosurgical tissue effect may be compromised. The overall surface area of the return electrode 360 is reduced. When the overall surface area of the return electrode 360 begins to approximate that of the active electrode 470, a tissue effect, which may include a plasma, may inadvertently fire on the return electrode 360. The distance between the active and return electrodes may also be non-uniform around the periphery of the active electrode 470. When the active electrode 470 is unevenly spaced from the return electrode 360 at one end than the other, the tissue effect may only treat tissue along some sides of the active electrode 470. The closer the active and return electrodes are, the higher the current densities. High current densities make it easier to form plasma on the active electrode. However, if the spacing between the active and return is larger between some peripheral portions of the active electrode 470 than others, the current densities are poor in those areas and plasma is less likely to form around that corresponding portion. In some cases, the plasma may only form along some portions, which may damage the electrodes or form too aggressive a tissue effect. If the overall distance between the return and active electrodes peripheries are too far, the firing the plasma become inconsistent or requires higher voltage inputs to achieve. Therefore, the balance between a spacer 480 for a stronger bond with the sleeve 310 and a reliable uniform electrosurgical tissue effect is preferred.

Like clip 390, distal end 400 includes a collar 490 that wraps around the external surface of the spacer 480 at a proximal end of spacer 480. Collar 490 may extend 360 degrees about a transverse cross section of distal end 400. This not only provides supplemental securement of a proximal portion of the spacer 380 but may also act as a return electrode 360. This collar 490 adds a securement between the sleeve 310 and spacer 480, avoiding interference with the inner rotating sleeve 350. The collar 490 may increase attachment of the spacer 480 to the outer sleeve, such that it can withstand clinically relevant loading during a procedure without compromise to the integrity of any part of the device. Collar 490 may extend around the distal end 400 and be welded 480 to the outer sleeve 310 to secure it in place and form a consistent electrical communication with outer sleeve 310. Collar 490 provides mechanical retention of the spacer 480. Spacer 480 is therefore sandwiched circumferentially between the outer sleeve 310 and collar 490. Spacer 480 may include an outer annular recess configured to nest at least a portion of collar 490. Collar 490 may be spaced proximally from the window 320 and active electrode 470. Collar 490 may be between 0.0020 and 0.0040 inches thick.

Regarding the electrosurgical tissue effect, collar 490, similar to clip 390, may be electrically conductive and may be in electrical communication with the outer sleeve 310 and thereby contribute towards the effective surface area of the return electrode 360. Collar 490 may effectively move a periphery of the return electrode 360 closer to the proximal end of the active electrode 470, and thereby forming a resultant return electrode periphery that more uniformly encircles the active electrode 470. Stated another way, the spacing between the active electrode 470 and return electrode 360 is made more uniform around more sides of the active electrode 470, because of the added collar 490. The collar 490 is tightly wrapped around the spacer 480 and outer sleeve 310 and may be include a weld 480 to fixedly secure the collar 490 to outer sleeve 310. Example locations of weld 480 are shown. Another example may include a circumferential laser weld around mating portions of the sleeve 310 and collar 490. Electrical communication between outer sleeve portion of the return electrode 360 and the collar 490 may be through the weld 480 and surface contact,

FIG. 5A-5G illustrates another distal end embodiment 500, similar to previously disclosed embodiments. Similar elements are given the same numerical indicator as distal end 300 and 400. Distal end 500 includes an outer sleeve 510, with an inner sleeve 350 coaxially disposed therein. Inner sleeve 350 may rotate relative to outer sleeve 510 to mechanically resect tissue via laterally disposed windows. Outer sleeve 510 is at least partially covered by insulative spacer 580, and spacer 580 carries an active electrode 570. A return electrode 360 may include a plurality of components or bodies with exposed electrically conductive surfaces and in electrical communication with each other. These components may include the exposed electrically conductive surface of inner sleeve 350, the exposed electrically conductive surface outer sleeve 510 and the externally exposed electrically conductive surface of a retention clip 590.

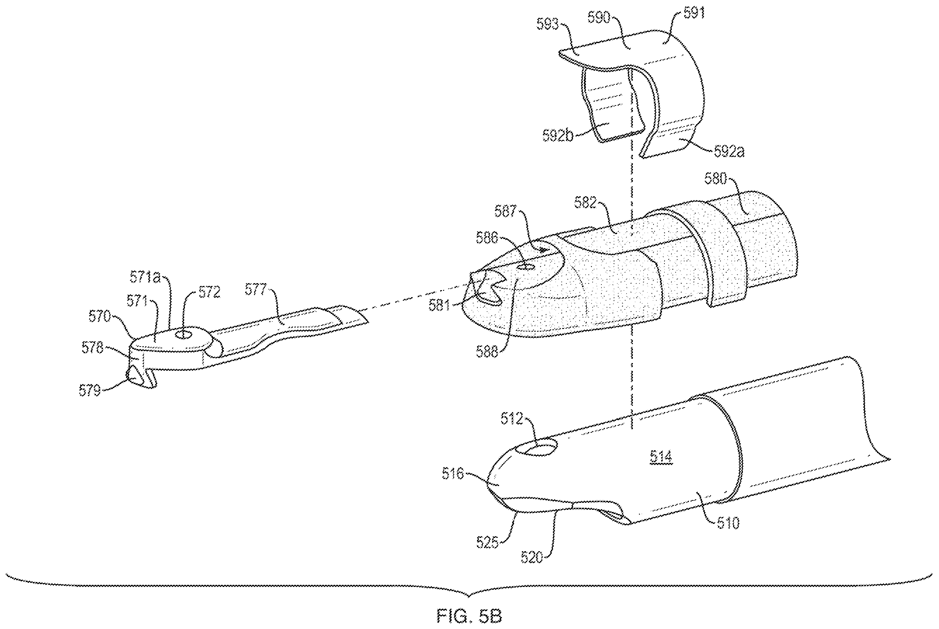

An exploded view of the components of distal end 500 is illustrated in FIG. 5B, with the inner sleeve 350 removed for simplification of the figure. Outer sleeve 510 defines an elongate sleeve with a cutting window 520 on a first circumferential side of distal end 500. Outer sleeve 510 extends proximally and may fixedly couple to hub, configured to operatively couple with handle end that may include the MDU 112. Outer sleeve 510 includes an aspiration aperture 512 through the outer sleeve wall thickness on a second, opposing circumferential side to the window 520. Aperture 512 may communicate with a corresponding aperture through active electrode 570 to aspirate fluid and debris therethrough while treating tissue. Distal end of outer sleeve 510 may define a reduced outer diameter portion 514 formed by reducing a thickness of the sleeve wall. This portion 514 defines a circumferential surface that engages an inner surface of insulative spacer 580, the reduced outer diameter portion reduced in diameter to act as a recess and maintain a smaller overall profile of the distal end 500. Outer sleeve 510 extends up to around tip 516 that curves towards longitudinal axis of sleeve 510, the curve extending up to and including bevelled cutting edge 525 of window 520.

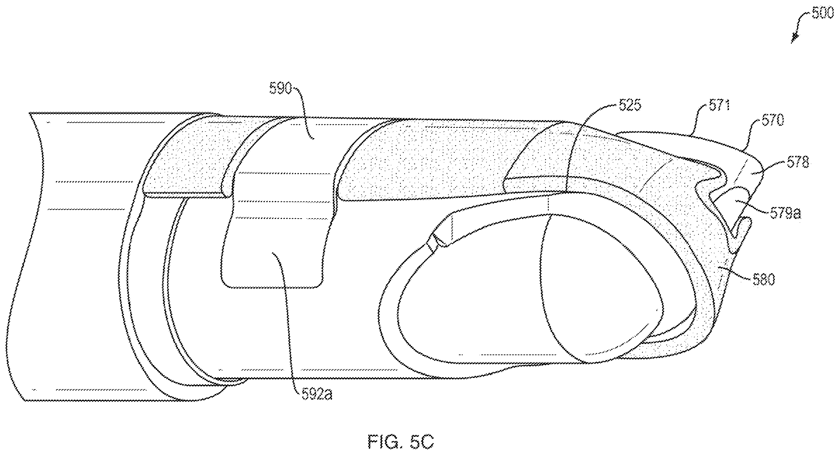

Spacer 580 is sized to mate with at least the reduced diameter portion 514 of outer sleeve 510 and covers an opposing circumferential side of sleeve 510 to window 520. Spacer 580 extends over and fixedly couples to a distal tip 516 of sleeve. Spacer 580 may define a unibody, and may have a contoured inner surface, contoured to match and directly engage the outer surface of sleeve 510, for a mating surface-to-surface contact between the two. Spacer 580 inner surface may be adhesively coupled to outer sleeve 510. Extending the spacer around the tip 516 spaces the active electrode 570 from the sleeve 510 and thereby the return electrode 360 to reduce inadvertent plasma formation on the sleeve 510. As such, spacer 580 defines a distal facing surface at a distal-most end of distal end 500. Securement between the spacer 580 and sleeve 510 around the distal tip 516 may be particularly relevant during use, as the very distal tip may be used to elevate and dissect tissue, putting a lifting load on the active electrode 570 and spacer 580. Having the spacer 580 wrap around the distal tip 516 improves securement of spacer 580 to sleeve 510. Spacer 580 may extend around the sleeve 510 up to the bevelled cutting edge boundary 525 of sleeve window 520. Spacer 580 may encircle at least a top circumferential half of sleeve 510. Stated another way and best illustrated in FIG. 5C and FIG. 5D, spacer 580 may cover at least half the circumference of the sleeve 510 at the distal end 500. Having the spacer 580 wrap around the sleeve 510 not only increases the surface area available for bonding between the sleeve 510 and spacer 580, but also forms a more equivalent spacing between proximal and distal edges of the active electrode 570 and return electrode 360. Spacing G1 and G2 are substantially equivalent to each other, illustrated in FIG. 5D. In addition, the inventors have found that in use, a predominant force on the device distal end is from a distal-most end in a proximal direction (F), acting to lift the spacer 580 and/or active electrode 570 up and thereby act to separate active electrode 570 and or spacer 580 from the sleeve 510. Spacer 580 preferably extends over and is bonded to a distal facing surface of sleeve 510.

Spacer 580 includes a plurality of recesses for nesting and securing active electrode 570 and clip 590. Retention clip 590 may encircle the spacer 580 and lie within a notch or recess 582 formed on outer surface of spacer 580. Clip 590 may be a stainless steel stamped and formed clip that fits around spacer 580. Recess 582 may mirror the profile of clip 590 and may help form a smoother and more continuous external surface of the distal end 500, thereby eliminating or diminishing snagging points therealong. Clip 590 may be welded to outer sleeve 510 to retain and electrically couple the clip 580 thereto. As explained herein, clip 590 provides a supplemental fixation between the spacer 580 and sleeve 510. Clip 590 may define a body portion 591 and free ends 592a, 592b. Free ends 592a, 592b may be shaped and radially jogged inward relative to the body portion 591. This radial jog preferably follows the contours of spacer 580, and the transition from the spacer 580 to the sleeve 510. The free ends 592a, 592b are preferably fixedly coupled to outer sleeve 510. Body 591 may define a distally extending tab 593 configured to form a more uniform spacing between active electrode 570 and return electrode 360. Tab 593 may extend distally towards the active electrode 570 on the same circumferential side of the sleeve 510 as the active electrode 570. Tab 593 may define a circumferential width equivalent to a corresponding active electrode circumferential width. Without the tab 593, the spacing between the active electrode 570 and closest portion of return electrode 360 may be significantly uneven around the active electrode 570, biased towards the distal end of active electrode 570. This may strongly bias the plasma formation and tissue effect towards the distal tip of the active electrode 570 and frustrate an even and controlled electrosurgical tissue effect, as explained earlier. Tab 593 has a lateral or circumferential extent and may define a distal most edge that encircles or approximates the proximal edge of the active electrode 570. The addition of clip 590 also increases an overall exposed surface area of return electrode 360 that may contact the electrically conductive fluid, mitigating any inadvertent tissue effect at the return electrode 360. For example, the clip 580 may increase the surface area of effective return electrode 360 by up to 50% and define the active electrode 570 to return surface area ratio closer to 1:7.

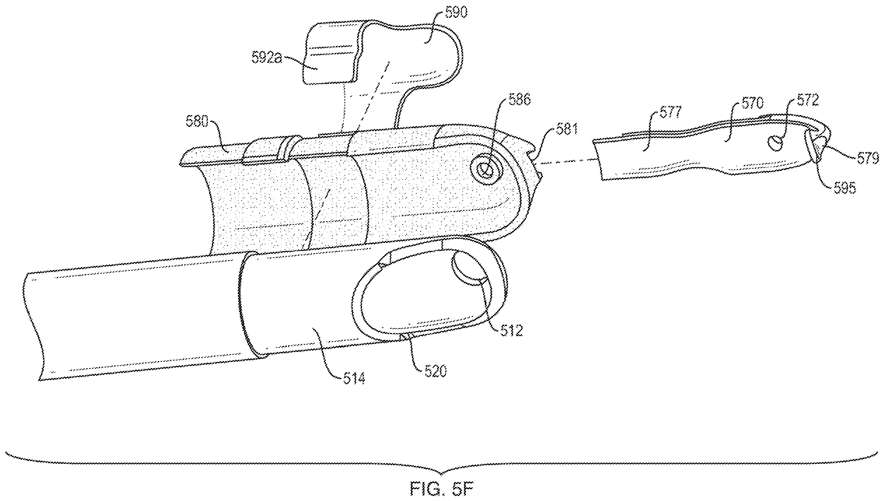

Spacer 580 includes a fluid aspiration aperture 586 in fluid communication and overlapping a corresponding aperture 512 through sleeve 510 and active electrode aperture 572. All three apertures communicate to remove tissue debris and fluid from the target site and along the device that may be coupled to a vacuum source for example. Spacer aperture 586 may define a bossed aperture (best seen in FIG. 5F). Boss may extend through sleeve aperture 512 to increase an insulated spacing between the active and return electrodes, inhibiting plasma firing through apertures (512, 572, 586) to the inner sleeve or outer sleeve.

Spacer 580 also nests and retains active electrode 570 in at least two discrete locations. The two locations may be axially separated by spacer circumferential surface 588. A first retaining location includes an axial tunnel 587 that houses and covers an active electrode leg 577. Axial tunnel 587 may slidingly fit with leg 577, thereby retaining the active electrode 570 proximal end. Adhesive may be inserted into and along tunnel 587 to improve retention and limit fluid ingress therealong. A proximal most end of leg 577 may electrically couple to wiring that extends along the device (not shown).

Extending distally from tunnel 587 is a circumferential surface 588, configured to support a circumferential portion 571 of active electrode 570. Active electrode 570 is a single body with a circumferential portion 571 and second electrode portion 578. Circumferential portion 571 may define a first treatment surface 571a of active electrode 860 disposed on a circumferential side of the distal end 500, opposite the window 520. The first treatment surface 571a may match the external contours of distal end 500. First treatment surface 571a may taper to a narrower distal end for finer dissection. Proximal edge 573 of treatment surface 571a is preferable axially spaced from a corresponding distal edge 584 of spacer 580. This axial space provides a relief as the active electrode 580 bends with use. Without this spacing proximal edge 583 may place damaging stresses on the spacer edge 584.

Spacer 580 also retains active electrode 570 at a distal location, axially separated from the proximal retention location (leg 577). Active electrode distal end includes second electrode portion 578 that is angularly offset from the first portion 571, and angularly offset from a longitudinal axis of the distal end 500. Second electrode portion 578 may define a distal facing treatment surface 579 that extends across the distal-most end of distal end 500. Second electrode portion 578 has a radial extent. Surface 579 may further include a sloped portion 579a configured to deflect forces (F) during treatment that may separate or peel the active electrode 570 from spacer 580. Slope 579a defines a concave surface that is distal facing. Slope 579a may taper to extend proximally as it extends towards the longitudinal axis. Treatment surface 579 generally enhances the performance of the tip of active electrode 570. It extends the functional base of the distal tip of active electrode 570.

Second electrode portion 578 extends from a distal end of first treatment portion 571 and may define a hook shape. Second electrode portion 578 may engage mating surfaces of a spacer recess 581. Spacer recess 581 may extend from a distal-most surface of spacer 580. Second electrode portion 578 may have an extent with at least one lateral flange 595 or protrusion disposed at an end of this extent. Illustrated FIG. 5E are a pair of opposing lateral flanges 595a, 595b, defining a triangular shape. A matching recess 581 in spacer 580 may receive and retain the opposing lateral flanges 595a, 595b. Second electrode portion 578 defines the second discrete retaining portion. Notch or recess 581 may define opposing groove(s) to receive the opposing lateral flanges 595a, 595b. As explained earlier, when loaded at the distal tip (F), the electrode 570 may tend to rotate about the electrode neck 577. If allowed to rotate significantly it can result in permanent offset of the electrode 570 or fatigue failure of the neck. This second retaining location at the distal-most end of the electrode 570 helps to mitigate this failure mode.

The flange(s) and matching groove(s) are configured to engage each other and secure electrode distal end with spacer. FIG. 6 and FIG. 7 illustrate other example embodiments with alternative flange and groove shapes, FIG. 6 illustrates a distal view of a distal end 600, wherein the active electrode 670 includes a second electrode portion 670 defining a distal-most end of distal end 600. Second electrode portion 670 includes "T" shaped bilateral flanges 695a, 695b. Spacer 680 includes matching grooves 685a, 685b, configured to engage the bilateral flanges 695a, 695b and secure distal end of electrode 670 with spacer 680. FIG. 7 illustrates a distal view of example embodiment distal end 700, wherein the active electrode 770 includes a second electrode portion 775 at a distal-most end of distal end 700. Second electrode portion 775 includes curved bilateral flanges 795a, 795b.

A further embodiment is shown in FIG. 8A and 8B, illustrating a distal end 800 with an active electrode 870 and spacer 860 only for simplicity of understanding. The active electrode 870 includes bilateral rails 875a, 875b along lateral edges of active electrode 870 that operatively engage bilateral slots along the spacer 860. While this embodiment offers a longer length of engagement, it may require an increase in cross sectional area of the distal end 800, creating a larger profile end compared with distal end 500 with a distal retention hooked end.

Another example embodiment of a distal end 900 is illustrated in FIGS. 9A and 9B. Like components are given the same numerical labels. In this embodiment, the collar 990, which may act in a similar manner to at least clip 390, 590 or collar 490, and may encircle the spacer 980. Collar 990 may include two axially extending tabs 995a, 995b. Each tab 995a, 995b may extend along lateral sides relative to active electrode 570, to surround a greater periphery of the active electrode 510. Spacer 980 may include a corresponding notch or recess (not shown) for receiving collar 990 therein. This recess may match the shape of collar 990 and may help form a smoother and more continuous external surface, thereby eliminating or diminishing snagging points therealong. Collar 990 may be like clip 380 or 590 or collar 490 in material and formation. Collar 990 may be welded to outer sleeve 910 to retain and electrically couple the collar 990 thereto. The spacer 980 may be adhesively coupled to outer sleeve 510, the collar 990 providing supplemental fixation between the spacer 980 and sleeve 910. The two distally extending tabs 995a, 995b are configured to improve a spacing between the active electrode 570 and return electrode. As explained herein, this may improve the electrosurgical tissue effect, as explained earlier. The addition of the collar 990 also increases an overall surface area of return electrode, more so than clip 590. For example, collar 990 may increase the surface area by up to 100% and keep the active electrode 570 to return surface area ratio closer to 1:10.

FIG. 10A-10E illustrates another example embodiment distal end 1000 of a combination tissue resecting probe, configured to function in a similar manner as previously disclosed embodiments, and therefore includes similar components. FIG. 10A illustrates the spacer and active electrode only for simplicity of understanding. Distal end 1000 includes an active electrode 1050 coupled to a spacer 1060, in a similar manner to electrode 570 and spacer 580. Active electrode 1050 includes a first treatment portion 1052 that defines an external circumferential surface of the distal end 1000. First treatment surface may taper as it extends distally to a finer tipped distal end for finer tissue dissection. Active electrode first treatment portion 1052 may include an aspiration aperture 1055 therethrough in fluid communication with an aspiration conduit that extends along the probe shaft, similar to previous embodiments. Active electrode 1050 also includes a distal hook 1056 extending towards the longitudinal axis of the device distal end 1000. Distal hook 1056 has a distal facing surface 1057 that may electrosurgically treat tissue at the very distal tip of the probe. Distal facing surface 1057 may have a lateral extent, more squared off than hook the distal facing surface 578. This may add structural integrity to distal hook 1056. Distal hook 1056 is configured to secure the active electrode 1050 with the spacer 1080 at the distal-most end.

FIG. 10B illustrates an exploded view of components of distal end 1000, and includes an inner sleeve 310, outer sleeve 1010 that may define a portion of return electrode 360, insulative spacer 1060, clip 1090 that also may define a portion of return electrode 360 and active electrode 1050. Embodiment 1000 is similar to embodiment 500 for the most part, except where notes. For example, in embodiment 1000, distal hook 1056 engages spacer 1060 with a proximally extending flange 1059 at the distal end. The spacer 1060 has a corresponding cavity 1062 and notch 1064, for receiving and engaging with hook 1056, shown in more detail in FIG. 10E.

Shown in cross section in FIG. 10E, proximally extending flange or projection 1059 is shown. Spacer 1060 includes a contoured cavity 1062 extending up to and including a distal-most surface of the spacer 1060, the cavity 1062 configured to receive distal hook 1056 therein and engage the projection 1059. Distal hook 1056 is recessed at least partly within cavity 1062, to secure the active electrode 1050, while allowing the surface 1057 access to tissue at distal tip of probe. Cavity 1062 includes a notch 1064 extending proximally further into cavity, configured to receive projection 1059. Notch 1064 is configured to receive and engage projection 1059 to secure distal end of active electrode 1050 and mechanically resist separation of the active electrode 1050 from the spacer 1060 during use of the probe. Notch 1064 and cavity 1062 are also configured to limit an axial location of the active electrode portion 1052. Notch 1064 and cavity 1062 cooperate with the active electrode 1050 to space a proximal edge 1053 of the active electrode treatment portion 1052 a predetermined axial distance from a spacer distal edge 1066. During assembly, active electrode 1050 may be slid proximally along the spacer 1060 to lie flat along spacer. Active electrode 1050 slides up until projection 1059 is disposed within cavity 1062 and engages a proximal surface of notch 1064. Active electrode 1050 may then be fixed in place, via adhesive for example. This location defines an axial spacing between the edges 1066 and 1053 configured to avoid transferring stresses to the spacer 1060 via bending and flexing of the active electrode during use. During use of the device, normal conditions may bend and flex the distal end 1000. FIG. 10C illustrates distal end 1000 top side, showing tapering active electrode portion 1052, that includes squared off distal facing surface 1057a.

The foregoing examples are to be considered in all respects illustrative rather than limiting of the disclosure described herein. Scope of the invention is defined by the appended claims, rather than by the foregoing description, and all changes that come within the meaning of the claims are therefore intended to be embraced therein.

## Claims

1. A tissue resecting probe for mechanically resecting and electrosurgically treating tissue comprising:
a tubular distal end that is electrically conductive, with a cutting window (520) on a first circumferential side;
an electrically insulative spacer (580) wrapped over a second circumferential side of the tubular distal end;
an active electrode (570) secured to the insulative spacer (580), the active electrode (570) defining two portions, angularly offset from each other, the two portions including a first portion (571) that extends circumferentially and axially along the tubular distal end and a second portion (578) that extends from a distal end of the first portion and across the tubular distal end (500), the second portion (578) having at least one flange (595) configured to engage a notch (581) in the electrically insulative spacer (580) to resist separation of the active electrode (570) from the electrically insulative spacer (580) and wherein the second portion (578) defines a distal-most surface of the tissue resecting probe.

2. The tissue resecting probe of claim 1 wherein the second portion (578) extends perpendicularly to the longitudinal axis.

3. The tissue resecting probe of claim 1 wherein the first portion (571) is axially tapered, configured to finely dissect tissue at the first portion distal end.

4. The tissue resecting probe of claim 1 wherein the at least one flange extends proximally into the electrically insulative spacer (580).

5. The tissue resecting probe of claim 1 wherein the at least one flange includes bilateral flanges.

6. The tissue resecting probe of claim 1 wherein the second portion (578) defines a distal facing concave surface configured to deflect external forces on the active electrode (570) during use.

7. The tissue resecting probe of claim 1 wherein the insulative spacer (580) extends around the tubular distal end up to a cutting edge of the cutting window.

8. The tissue resecting probe of claim 1 wherein the insulative spacer (580) extends over to cover a distal facing end surface of the tubular distal end, thereby defining at least a portion of a distal-most end of the tissue resecting probe.

9. The tissue resecting probe of claim 1 wherein the active electrode (570) includes a neck extending proximally from the active electrode first portion (571) configured to extend along a tunnel (587) through the insulative spacer (580) and thereby retain the active electrode (570) at a second location proximally separated from the second portion (578).

10. The tissue resecting probe of claim 1 further comprising a retention clip (590) extending around an external surface of insulative spacer (580) and both electrically and mechanically coupled to the tubular distal end.

11. The tissue resecting probe of claim 10 wherein the tubular distal end and retention clip (590) both define external surfaces of the tissue resecting probe.

12. The tissue resecting probe of claims 11 wherein the retention clip (590) includes a distally extending tab disposed adjacent the active electrode (570), configured to arrange a proximal end of the active electrode (570) and the retention clip (590) according to a target spacing and thereby improve the electrosurgical tissue effect.

13. The tissue resecting probe of claim 10 wherein retention clip (590) includes bilateral radially jogged ends (592a, 592b).

14. The tissue resecting probe of claim 1 wherein the at least one flange is configured to engage the notch and concomitantly axially position the active electrode (570) to maintain an axial gap between the insulative spacer (580) and a proximal end of the active electrode (570), the axial gap configured to mitigate stress concentrations along the tubular distal end during use.

## Patentansprüche

1. Eine Geweberesektionssonde zur mechanischen Resektion und elektrochirurgischen Behandlung von Gewebe, die Folgendes umfasst:
ein röhrenförmiges distales Ende, das elektrisch leitend ist, mit einem Schneidfenster (520) auf einer ersten Umfangsseite;
einen elektrisch isolierenden Abstandshalter (580), der über eine zweite Umfangsseite des röhrenförmigen distalen Endes gewickelt ist;
eine aktive Elektrode (570), die an dem isolierenden Abstandshalter (580) befestigt ist, wobei die aktive Elektrode (570) zwei voneinander winkelversetzte Abschnitte definiert, wobei die zwei Abschnitte einen ersten Abschnitt (571), der sich umlaufend und axial entlang des röhrenförmigen distalen Endes erstreckt, und einen zweiten Abschnitt (578), der sich aus einem distalen Ende des ersten Abschnitts und über das röhrenförmige distale Ende (500) hinweg erstreckt, einschließen, wobei der zweite Abschnitt (578) mindestens einen Flansch (595) aufweist, der zur Eingriffnahme mit einer Kerbe (581) in dem elektrisch isolierenden Abstandshalter (580) ausgelegt ist, um einer Trennung der aktiven Elektrode (570) von dem elektrisch isolierenden Abstandshalter (580) zu widerstehen, und wobei der zweite Abschnitt (578) eine distalste Oberfläche der Geweberesektionssonde definiert.

2. Geweberesektionssonde nach Anspruch 1, wobei sich der zweite Abschnitt (578) senkrecht zu der Längsachse erstreckt.

3. Geweberesektionssonde nach Anspruch 1, wobei der erste Abschnitt (571) axial verjüngt ist, dazu ausgelegt, Gewebe an dem distalen Ende des ersten Abschnitts fein zu sezieren.

4. Geweberesektionssonde nach Anspruch 1, wobei sich der mindestens eine Flansch proximal in den elektrisch isolierenden Abstandshalter (580) hinein erstreckt.

5. Geweberesektionssonde nach Anspruch 1, wobei der mindestens eine Flansch bilaterale Flansche einschließt.

6. Geweberesektionssonde nach Anspruch 1, wobei der zweite Abschnitt (578) eine distal gerichtete konkave Oberfläche einschließt, die dazu ausgelegt ist, während der Verwendung auf die aktive Elektrode (570) einwirkende äußere Kräfte abzulenken.

7. Geweberesektionssonde nach Anspruch 1, wobei sich der isolierende Abstandshalter (580) um das röhrenförmige distale Ende bis hin zu einer Schneidkante des Schneidfensters erstreckt.

8. Geweberesektionssonde nach Anspruch 1, wobei sich der isolierende Abstandshalter (580) hinüber erstreckt, um eine distal gerichtete Endfläche des röhrenförmigen distalen Endes abzudecken, wodurch mindestens ein Abschnitt eines distalsten Endes der Geweberesektionssonde definiert wird.

9. Geweberesektionssonde nach Anspruch 1, wobei die aktive Elektrode (570) einen Hals einschließt, der sich proximal aus dem ersten Abschnitt (571) der aktiven Elektrode erstreckt, der dazu ausgelegt ist, sich entlang eines Tunnels (587) durch den isolierenden Abstandshalter (580) zu erstrecken und dadurch die aktive Elektrode (570) an einem zweiten Ort festzuhalten, der proximal von dem zweiten Abschnitt (578) getrennt ist.

10. Geweberesektionssonde nach Anspruch 1, die ferner eine Halteklammer (590) umfasst, die sich um eine Außenfläche des isolierenden Abstandshalters (580) herum erstreckt und sowohl elektrisch als auch mechanisch mit dem röhrenförmigen distalen Ende gekoppelt ist.

11. Geweberesektionssonde nach Anspruch 10, wobei beide von dem röhrenförmigen distalen Ende und der Halteklammer (590) Außenflächen der Geweberesektionssonde definieren.

12. Geweberesektionssonde nach Anspruch 11, wobei die Halteklammer (590) eine sich distal erstreckende Lasche einschließt, die sich neben der aktiven Elektrode (570) befindet, die dazu ausgelegt ist, ein proximales Ende der aktiven Elektrode (570) und die Halteklammer (590) gemäß einem Zielabstand anzuordnen und dadurch den elektrochirurgischen Gewebeeffekt zu verbessern.

13. Geweberesektionssonde nach Anspruch 10, wobei die Halteklammer (590) bilaterale radial abgestufte Enden (592a, 592b) einschließt.

14. Geweberesektionssonde nach Anspruch 1, wobei der mindestens eine Flansch dazu ausgelegt ist, mit der Kerbe in Eingriff zu kommen und gleichzeitig die aktive Elektrode (570) axial zu positionieren, um einen axialen Spalt zwischen dem isolierenden Abstandshalter (580) und einem proximalen Ende der aktiven Elektrode (570) aufrechtzuerhalten, wobei der axiale Spalt dazu ausgelegt ist, während der Verwendung Spannungsspitzen entlang des röhrenförmigen distalen Endes abzuschwächen.

## Revendications

1. Sonde de résection tissulaire conçue pour une résection mécanique et un traitement électrochirurgical de tissus, comprenant :
une extrémité distale tubulaire qui est électroconductrice, pourvue d'une fenêtre de coupe (520) sur un premier côté circonférentiel ;
un espaceur électro-isolant (580) enroulé par-dessus un deuxième côté circonférentiel de l'extrémité distale tubulaire ;
une électrode active (570) fixée à l'espaceur isolant (580), l'électrode active (570) délimitant deux parties, décalées angulairement l'une par rapport à l'autre, les deux parties comprenant une première partie (571) qui s'étend circonférentiellement et axialement le long de l'extrémité distale tubulaire et une deuxième partie (578) qui s'étend depuis une extrémité distale de la première partie et à travers l'extrémité distale tubulaire (500), la deuxième partie (578) comportant au moins une bride (595) configurée pour s'introduire dans une encoche (581) dans l'espaceur électro-isolant (580) afin de résister à une séparation de l'électrode active (570) de l'espaceur électro-isolant (580) et dans laquelle la deuxième partie (578) délimite une surface la plus distale de la sonde de résection tissulaire.

2. Sonde de résection tissulaire selon la revendication 1, dans laquelle la deuxième partie (578) s'étend perpendiculairement à l'axe longitudinal.

3. Sonde de résection tissulaire selon la revendication 1, dans laquelle la première partie (571) est effilée axialement et configurée pour disséquer finement le tissu à l'extrémité distale de la première partie.

4. Sonde de résection tissulaire selon la revendication 1, dans laquelle l'au moins une bride s'étend proximalement dans l'espaceur électro-isolant (580).

5. Sonde de résection tissulaire selon la revendication 1, dans laquelle l'au moins une bride comprend des brides bilatérales.

6. Sonde de résection tissulaire selon la revendication 1, dans laquelle la deuxième partie (578) délimite une surface concave orientée distalement et configurée pour dévier des forces externes exercées sur l'électrode active (570) en cours d'utilisation.

7. Sonde de résection tissulaire selon la revendication 1, dans laquelle l'espaceur isolant (580) s'étend autour de l'extrémité distale tubulaire jusqu'à un bord de coupe de la fenêtre de coupe.

8. Sonde de résection tissulaire selon la revendication 1, dans laquelle l'espaceur isolant (580) s'étend, pour la recouvrir, par-dessus une surface d'extrémité orientée distalement de l'extrémité distale tubulaire, en délimitant ainsi au moins une partie d'une extrémité la plus distale de la sonde de résection tissulaire.

9. Sonde de résection tissulaire selon la revendication 1, dans laquelle l'électrode active (570) comprend un col s'étendant proximalement depuis la première partie (571) de l'électrode active, configuré pour s'étendre le long d'un tunnel (587) à travers l'espaceur isolant (580) et ainsi retenir l'électrode active (570) à un deuxième emplacement, séparé proximalement de la deuxième partie (578).

10. Sonde de résection tissulaire selon la revendication 1, comprenant en outre une pince de rétention (590) s'étendant autour d'une surface externe de l'espaceur isolant (580) et couplée aussi bien électriquement que mécaniquement à l'extrémité distale tubulaire.

11. Sonde de résection tissulaire selon la revendication 10, dans laquelle l'extrémité distale tubulaire et la pince de rétention (590) délimitent toutes deux des surfaces externes de la sonde de résection tissulaire.

12. Sonde de résection tissulaire selon la revendication 11, dans laquelle la pince de rétention (590) comprend une languette s'étendant distalement, disposée de façon adjacente à l'électrode active (570), configurée pour placer une extrémité proximale de l'électrode active (570) et la pince de rétention (590) selon un espacement cible, en améliorant ainsi l'effet électrochirurgical sur le tissu.

13. Sonde de résection tissulaire selon la revendication 10, dans laquelle la pince de rétention (590) comprend des extrémités bilatérales décalées radialement (592a, 592b).

14. Sonde de résection tissulaire selon la revendication 1, dans laquelle l'au moins une bride est configurée pour s'introduire dans l'encoche et simultanément positionner axialement l'électrode active (570) afin de maintenir un espace axial entre l'espaceur isolant (580) et une extrémité proximale de l'électrode active (570), l'espace axial étant configuré pour atténuer des concentrations de contrainte le long de l'extrémité distale tubulaire en cours d'utilisation.
